(19) Europäisches Patentamt
European Patent Office
Office européen des brevets

(11) **EP 3 526 796 B1**

(12) **EUROPÄISCHE PATENTSCHRIFT**

(45) Veröffentlichungstag und Bekanntmachung des Hinweises auf die Patenterteilung:
**30.06.2021 Patentblatt 2021/26**

(21) Anmeldenummer: **17790975.1**

(22) Anmeldetag: **09.10.2017**

(51) Int Cl.:
*G11B 27/031* *(2006.01)*         *H04N 5/222* *(2006.01)*
*G06T 7/143* *(2017.01)*         *G06T 7/11* *(2017.01)*
*G06T 7/194* *(2017.01)*         *G16B 20/00* *(2019.01)*

(86) Internationale Anmeldenummer:
**PCT/EP2017/075624**

(87) Internationale Veröffentlichungsnummer:
**WO 2018/069219 (19.04.2018 Gazette 2018/16)**

(54) **SYSTEM ZUR DYNAMISCHEN KONTRASTMAXIMIERUNG ZWISCHEN VORDERGRUND UND HINTERGRUND IN BILDERN ODER/UND BILDSEQUENZEN**

SYSTEM FOR DYNAMICALLY MAXIMIZING THE CONTRAST BETWEEN THE FOREGROUND AND BACKGROUND IN IMAGES AND/OR IMAGE SEQUENCES

SYSTÈME DE MAXIMISATION DE CONTRASTE DYNAMIQUE ENTRE LE PREMIER PLAN ET L'ARRIÈRE-PLAN DANS DES IMAGES ET/OU DES SÉQUENCES D'IMAGES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **14.10.2016 DE 102016119639**

(43) Veröffentlichungstag der Anmeldung:
**21.08.2019 Patentblatt 2019/34**

(73) Patentinhaber: **uniqFEED AG**
**8005 Zürich (CH)**

(72) Erfinder: **DRAGON, Ralf**
**30559 Hannover (DE)**

(74) Vertreter: **Müller Hoffmann & Partner**
**Patentanwälte mbB**
**St.-Martin-Strasse 58**
**81541 München (DE)**

(56) Entgegenhaltungen:
**EP-A1- 2 463 821         FR-A1- 2 959 339**
**GB-A- 2 408 164         US-A1- 2003 001 954**
**US-A1- 2015 163 416         US-B1- 6 384 871**

**Beschreibung**

Die Erfindung betrifft ein System zur dynamischen Kontrastmaximierung zwischen Vordergrund und Hintergrund in Bildern oder/und Bildsequenzen.

Für die nahtlose Integration virtueller Objekte in reelle Szenen (auch Augmented Reality genannt) ist es bekannt, dass zwei Probleme zu lösen sind. Zum einen ist die Transformation zwischen Kamerakoordinatensystem und Szenenkoordinatensystem zu bestimmen und zum anderen sind Verdeckungen virtueller Objekte, die in der Regel im Hintergrund abgebildet sind, durch reelle Objekte, die in der Regel im Vordergrund sind, zu bestimmen.

Im Anwendungsgebiet Fernsehübertragung bzw. Broadcasting ist man daran interessiert, die beiden genannten Probleme möglichst nicht-mechanisch zu lösen, d.h., ohne den zusätzlichen Einsatz von Markern oder Sensoren. Dies erlaubt eine kürzere Aufbauzeit und reduziert die Komplexität eines insgesamt aufzubauenden Übertragungssystems vor Ort. Gerade bei Live-Übertragungen müssen der Großteil der Geräte zum Einsatzort transportiert und anschließend auf- und abgebaut werden.

Zur Lösung des Transformationsproblems gibt es einerseits hardware-basierte Lösungen wie beispielsweise "encoded heads" (z.B. von vinten.com), und andererseits software-basierte, visuell arbeitende Lösungen. Beide Lösungen stellen eine Abbildung x=M(X) bereit, die es erlaubt, eine 3D-Koordinate X eines virtuellen Objektes im 3D-Raum in eine Kamerakoordinate x im aktuellen Kamerabild abzubilden.

Bisher bekannte Segmentierungs-Methoden benötigen sogenannte Keying-Hinweise, wie z.B. eine bestimmte Hintergrundfarbe (z.B. bei Chroma Keying oder Infrarot-Keying). Diese mechanischen Lösungen haben jedoch die oben genannten Nachteile, da die Keying-Hinweise am Übertragungsort installiert werden müssen.

GB 2 408 164 A (BREWARD ALASTAIR [ES]) 18. Mai 2005 (2005-05-18) offenbart ein System, in dem ein LED-System (Displayvorrichtung) für Bannerwerbung dynamisch kontrolliert ist, so dass Anzeigeinformation auf dem LED-System angepasst wird, damit die Anzeigeinformation auf den durch eine Kamera aufgenommenen; Bildern sichtbar ist.

Aufgabe der Erfindung ist es, die obigen Nachteile zu vermeiden.

Zur Lösung dieser Aufgabe wird ein System zur dynamischen Kontrastmaximierung zwischen Vordergrund und Hintergrund in Bildern oder/und Bildsequenzen nach Anspruch 1 vorgeschlagen.

Durch die Berücksichtigung von Qualitätsdaten über die Segmentierung ist es möglich, eine Aussage darüber zu erhalten, ob Vordergrund und Hintergrund in ausreichendem Kontrast zueinander sind. Bei einem nicht ausreichenden Kontrast, kann die Anzeigeinformation angepasst werden, so dass bei unverändertem Vordergrund, ein kontrastreicher Hintergrund erzeugt werden kann.

Das System weist ferner eine Datenbank auf, die mit dem Kontrollmodul und der Displayvorrichtung verbunden ist, wobei die Datenbank dazu eingerichtet ist, eine Abfolge von Anzeigeinformationen zu speichern, die nacheinander auf der Displayvorrichtung darzustellen sind. Die Datenbank kann beispielsweise Teil eines Medienservers sein. Der Medienserver überträgt abzuspielende Anzeigeinformationen, beispielsweise Videoclips, an die Displayvorrichtung.

Hierbei ist es bevorzugt, dass das Kontrollmodul dazu eingerichtet ist, die gespeicherte Abfolge der Anzeigeinformationen zu verändern. In anderen Worten kann das Kontrollmodul den Medienserver anweisen, eine bestimmte Anzeigeinformation an der Displayvorrichtung auszugeben bzw., darzustellen. Um eine optimale Regelung mittels des Kontrollmoduls zu erreichen, kann die vom Medienserver an der Displayvorrichtung ausgegebene Anzeigeinformation (Videoclip) auch an das Kontrollmodul übermittelt werden.

Das Analysemodul ist dazu eingerichtet, bei der Segmentierung eine Energiefunktion anzuwenden und zu minimieren, wobei die Energiefunktion auf Wahrscheinlichkeitsverteilungen von Vordergrundobjekten und Hintergrundobjekten oder/und auf pixelweisen Wahrscheinlichkeitsverteilungen basiert. Die Segmentierung in Vorder- und Hintergrund (wobei auch Zwischenwerte wie Halbverdeckungen oder Unschärfen möglich sind) wird mit Y bezeichnet.

Hier kommen Segmentierungs-Methoden zum Einsatz, die keine Keying-Hinweise benötigen, sondern beliebige Vorder- und Hintergrundobjekte trennen können. Dabei wird eine Energiefunktion E(Y) definiert, die minimiert werden soll. E(Y) kann dem negativen Logarithmus der Wahrscheinlichkeit entsprechen, dass eine Verdeckung korrekt bestimmt wurde. Methoden der konvexen Optimierung erlauben es, die Energie effizient und optimal unter Berücksichtigung jedes einzelnen Bildpunktes zu minimieren, d.h., die entsprechende Wahrscheinlichkeit zu maximieren. Für die Minimierung der Energiefunktion E(Y) ist allerdings a-priori-Wissen über das Aussehen des Hintergrundes und des Vordergrundes nötig, insbesondere in Form von Wahrscheinlichkeitsverteilungen F (Vordergrund) und B (Hintergrund) bzw. pixelweisen Verteilungen $f_i$ und $b_i$, wobei i einen bestimmten Bildpunkt/Pixel bezeichnet.

Dieses a-priori Wissen muss aufgrund möglicher Helligkeitsänderungen oder Änderungen des Aussehens aktualisiert werden, was Drifting (das fortschreitende Abweichen eines Modells von der Realität) verursacht. Durch den oben beschriebene Rückkopplungsmechanismus, bei dem Qualitätsdaten über die Segmentierung an das Kontrollmodul übertragen werden und das Kontrollmodul die Anzeigeinformationen basierend auf den Qualitätsdaten anpasst, kann dem Drift-Problem entgegengewirkt werden. Dies erlaubt deswegen die Verwendung aktueller Segmentierungsmethoden mit verbesserter Leistung. Die Rückkopplung findet insbesondere über die Displayvorrichtung statt, vorzugsweise ein LED-System, wie es z.B. für Bannerwerbung bei vielen Sportereignissen vorhanden ist.

Das Kontrollmodul ist weiter dazu eingerichtet, für verbleibende Anzeigeinformationen der Abfolge zu bestimmen, bei welcher Anzeigeinformation sich eine erwartete Wahrscheinlichkeitsverteilung des Vordergrunds und eine erwartete Wahrscheinlichkeitsverteilung des Hintergrund der Bildaufnahmeeinrichtung am meisten unterscheiden.

Das Kontrollmodul kann auch dazu eingerichtet sein, für eine betreffende Bildaufnahmeeinrichtung eine Wahrscheinlichkeitsabbildung zu berechnen, wobei die Wahrscheinlichkeitsabbildung beschreibt, welche Wahrscheinlichkeitsverteilung in einem Bild der betreffenden Bildaufnahmeeinrichtung vorliegt, wenn die Displayvorrichtung eine der verbleibenden Anzeigeinformationen darstellt.

Das Kontrollmodul kann ferner dazu eingerichtet sein, die Wahrscheinlichkeitsabbildung basierend auf der zeitveränderlichen 2D-2D-Abbildung $x=F(d)$ zwischen d im Koordinatensystem der Displayvorrichtung und x im Koordinatensystem der Bildaufnahmeeinrichtung zu ermitteln. Diese 2D-2D-Abbildung F kann beispielsweise durch Anwenden von $x=M(X)$ bestimmt werden, wobei X die physische Position X des Display-Punktes d ist.

Das Kontrollmodul kann dazu eingerichtet sein, die Wahrscheinlichkeitsabbildung basierend auf einer radiometrischen Kalibrierung zwischen der Displayvorrichtung und der betreffenden Bildaufnahmeeinrichtung zu ermitteln.

An der Displayvorrichtung können mehrere beliebig gestaltete Kalibriermarken angeordnet sein, die von der Bildaufnahmeeinrichtung erfassbar sind, um die 2D-2D-Transformation F zwischen dem Koordinatensystem der Displayvorrichtung und dem Koordinatensystem der Bildaufnahmeeinrichtung zu erleichtern. Damit derartige Kalibriermarken nicht regelmäßig auf- und abgebaut werden müssen, können die Displayvorrichtungen solche Kalibriermarken dauerhaft aufweisen. Die Kalibriermarken können auch derart gestaltet sein, dass eine radiometrische Kalibrierung zwischen Aufnahmevorrichtung und Display möglich ist.

Das Kontrollmodul kann weiter dazu eingerichtet sein, in die Abfolge von Anzeigeinformationen Kalibrierinformation einzufügen, wobei die Displayvorrichtung dazu eingerichtet ist, die Kalibrierinformation darzustellen. Dabei kann die Kalibrierinformation wenigstens ein einzelnes einfarbiges Bild umfasst, wobei das Bild eine Darstelldauer von wenigstens einem oder genau einem Frame aufweist, und wobei das Bild vorzugsweise eine Farbe aufweist, die einen hohen Kontrast zu einem Vordergrundobjekt aufweist. Wird eine Kalibrierinformation als einfarbiges Bild lediglich für die Dauer eines Frames auf der Displayvorrichtung angezeigt, ist die Kalibrierinformation für einen Betrachter der Displayvorrichtung von bloßem Auge nicht erkennbar. Allerdings wird die an der Displayvorrichtung dargestellte Kalibrierinformation von der Bildaufnahmeeinrichtung aufgenommen, so dass der betreffende Frame dazu verwendet werden kann, Wissen über Vordergrundobjekte und Hintergrundobjekte zu aktualisieren, weil für diesen Frame bekannt ist, welche Farbe der Hintergrund aufweist.

Die oben genannte Aufgabe wird auch durch ein Verfahren zur dynamischen Kontrastmaximierung zwischen Vordergrund und Hintergrund in Bildern oder/und Bildsequenzen nach Anspruch 9 gelöst.

Dieses Verfahren kann weitere optionale Schritte umfassen entsprechend den oben für das System beschriebenen Merkmalen. Entsprechend können weitere optionale Aspekte des Verfahrens direkt aus diesen Merkmalen abgeleitet werden.

Nachfolgend wird die Erfindung unter Bezugnahme auf die anliegenden Figuren beispielhaft und nicht einschränkend beschrieben.

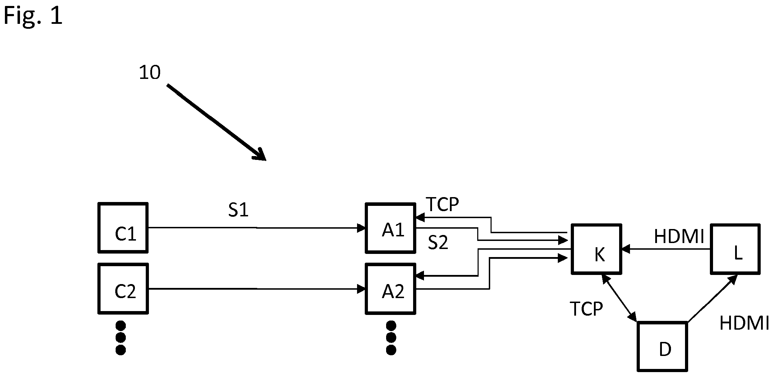

Fig. 1 zeigt ein schematisches Diagramm des hier vorgestellten Systems.

Ein System 10 zur dynamischen Kontrastmaximierung umfasst mehrere Bildaufnahmeeinrichtungen C1, C2, Ck (durch drei schwarze Punkte unterhalb von C2 angedeutet) und mehrere Analysemodule A1, A2 bis Ak (durch drei schwarze Punkte unterhalb von A2 angedeutet). Jeder Kamera C1, C2 ist ein Analysemdodul A1, A2 zugeordnet.

Die Kameras C1, C2, Ck leiten geeignete Daten S1k an das jeweilige Analysemodule A1, A2, Ak weiter. Die Analysemodule A1, A2, Ak bestimmen (1) die aktuelle 2D-2D-Abbildung F1, F2, Fk zwischen einer 2D-Koordinate im Displaykoordinatensystem von L in das Bildkoordinatensystem der jeweiligen Kamera C1, C2, Ck und (2) die bildpunktweise Verdeckung. Die Daten S1k sind je nach Analysemodul rein optisch, können jedoch auch Sensordaten von Schrittsensoren, Beschleunigungssensoren etc. beinhalten. Das Mapping Fk kann manuell kalibriert worden sein, oder mit einem automatischen Kalibrierverfahren.

Die Ausgangsdaten S2k der Analysemodule A1, A2, Ak beschreiben die bestimmten Mappings Fk sowie Metadaten über die Verdeckung. Diese Ausgangsdaten werden an ein Kontrollmodul K übertragen.

In die andere Richtung (angedeutet durch die Pfeile TCP) vom Kontrollmodul K zu den Analysemodulen Ak fließen Metainformationen zur Verbesserung der Segmentierung.

Ferner ist das Kontrollmodul K über ein Netzwerk verbunden mit einer Datenbank, D, in der Anzeigeinformationen (Videoclip) gespeichert sind, die auf dem LED-System L dargestellt werden können. Das Kontrollmodul hat insbesondere Kontroll-Zugriff über das Netzwerk auf einen Medienserver mit einer Videoclip-Datenbank D. Der Medienserver sendet abzuspielende Videoclips bzw. Anzeigeinformationen Z an das LED-System L.

Das Kontrollmodul K ist dazu eingerichtet, den Medienserver anzuweisen, bestimmte Anzeigeinformationen bzw. Videoclips Z über ein Bildtransportprotokoll BP, z.B. über HDMI, SDI, Displayport oder SDI over TCP, an die Displayvorrichtung L auszugeben. Zur Regelung wird das aktuell ausgegebene Videobild wiederum an K über BP zurückgegeben.

Zu beachten ist insbesondere der Aufbau, mit dem das Kontrollmodul K das Videomaterial (Anzeigeinformation) auswählt, dass auf der Displayvorrichtung L angezeigt werden soll, sowie der Regelkreis mit Feedback zur Kamera C1, C2. Üblicherweise ist das Videomaterial (Videoclips) a priori in einer Playlist organisiert, die während einer Übertragung eines Ereignisses, wie beispielsweise eines Spiels in einer bestimmten Sportart, ohne Möglichkeit auf Adaption abgespielt wird.

Vorliegende ist das Kontrollmodul K dazu eingerichtet, eine die Reihenfolge R von verbleibenden Anzeigeinformationen bzw. Videoclips Z dynamisch anzupassen, um möglichst gut Segmentierungsresultate zu erhalten.

Hierzu wird in den Metadaten S2k eine Metrik über die Qualität (Qualitätsdaten) der Segmentierung übertragen, beispielsweise die nach der Minimierung resultierende Segmentierungsenergie Ek(L). Überschreitet diese Energie ein akzeptables Maß in einer oder mehreren Kameras C1, C2, Ck, wird die Videoclip-Reihenfolge der verbleibenden Abfolge von Videoclips dynamisch angepasst. Wie bereits einleitend erwähnt kommen Segmentierungs-Methoden zum Einsatz, die keine Keying-Hinweise benötigen, sondern beliebige Vorder- und Hintergrundobjekte trennen können. Dabei wird eine Energiefunktion E(Y) definiert, die minimiert werden soll. E(Y) kann dem negativen Logarithmus der Wahrscheinlichkeit entsprechen, dass eine Verdeckung korrekt bestimmt wurde. Methoden der konvexen Optimierung erlauben es, die Energie effizient und optimal unter Berücksichtigung jedes einzelnen Bildpunktes zu minimieren, d.h., die entsprechende Wahrscheinlichkeit zu maximieren. Für die Minimierung der Energiefunktion E(Y) ist allerdings a-priori-Wissen über das Aussehen des Hintergrundes und des Vordergrundes nötig, insbesondere in Form von Wahrscheinlichkeitsverteilungen F (Vordergrund) und B (Hintergrund) bzw. pixelweisen Verteilungen f_i und b_i.

In der nachfolgenden Gleichung bezeichnet 1 das Analysemodul, bei dem die verbleibende Energie Ek(Yk) am größten ist, d.h., die Segmentierung am wahrscheinlichsten nicht korrekt bzw. am unsichersten ist. Dies ist ein Hinweis auf einen schlechten Kontrast zwischen Vordergrind und Hintergrund.

$$l = \arg\_k \max E_k(Y_k)$$

Entsprechend wird beispielsweise für die ausgewählte Kamera Cl die Abbildung Gl(Z) ermittelt, die beschreibt, welche Wahrscheinlichkeitsverteilung (an welchem Pixel) im Kamerabild von Cl vorliegt, wenn das LED-System L die Anzeigeinformation bzw. den Videoclip Z abspielt. Gl berücksichtigt das 2D-2D-Mapping Fl vom LED-System L zur Kamera Cl und Hintergrundinformationen einer radiometrischen Kalibrierung vom LED-System L zur Kamera Cl.

Für jeden möglichen in der Abfolge verbleibenden Videoclip Zm (Anzeigeinformation) wird die Abbildung Gl(Zm) bestimmt. Es wird derjenige Clip Zn als nächstes abgespielt, bei dem die Wahrscheinlichkeitsverteilung Fl des Vordergrundes von Kamera Cl und die erwartete Wahrscheinlichkeitsverteilung Gl des Hintergrundes von Kamera Cl sich am meisten unterscheiden:

$$n = \arg\_m \max d(Fl, Gl(Zm))$$

Eine Metrik d zum Vergleich von Vorder- und Hintergrundverteilung ist beispielsweise die Kullback-Leibler-Divergenz.

Das hier beschriebene System 10 und zugehörige Verfahrensschritte ermöglichen es, periodisch, d.h., wenn die verbleibende Energie Ek aufgrund ungenauem Wissens ansteigt, in jedem Kamerabild Wissen über Vorder- und Hintergrundaussehen zu aktualisieren und damit Drifting (das fortschreitende Abweichen eines Modells von der Realität) aktiv zu verhindern.

Die hier beschriebene Displayvorrichtung L kann ein LED-System sein, etwa in der Form von Werbebanden. LED-Systeme L weisen intern eine Ansteuerbox auf, die geeignete vorbereitete Videosignale über ein Bildtransportprotokoll, z. B. HDMI o.ä. entgegennimmt. Ferner weisen LED-Systeme eine Vielzahl von sogenannten Cabinets auf, die jeweils einen kleinen Teil des Gesamtbildes darstellen können und mit der Ansteuerbox verbunden sind. Die Ansteuerbox gibt dabei jedem Cabinet einen Bildausschnitt des ursprünglichen Videos weiter. Es kann zur Vereinfachung angenommen werden, dass ein LED-System einem regulären Bildschirm entspricht, nur dass der Aufbau eines LED-Systems deutlich flexibler gestaltet sein kann, z.B., dass kein rechteckiges Layout gewählt werden muss.

Es wird darauf hingewiesen, dass das hier beschriebene System 10 auch Teil eines übergeordneten Fernsehübertragungssystems sein kann, wie es in der von der gleichen Anmelderin am gleichen Tag eingereichten Anmeldung mit dem Titel "Fernsehübertragungssystem zur Erzeugung angereicherter Bilder" beschrieben ist. Das hier beschriebene Kontrollmodul kann beim dort beschriebenen System im Steuermodul 18 enthalten sein. Die hier beschriebene Displayvorrichtung kann beim dort beschriebenen LED-System 20 enthalten sein.

**Patentansprüche**

1. System zur dynamischen Kontrastmaximierung zwischen Vordergrund und Hintergrund in Bildern oder/und Bildsequenzen, umfassend

wenigstens eine Bildaufnahmeeinrichtung (C1, C2), wenigstens ein mit der Bildaufnahmeeinrichtung (C1, C2) verbundenes Analysemodul (A1, A2), das dazu eingerichtet ist, eine Transformation zwischen einem zweidimensionalen Koordinatensystem der Bildaufnahmeeinrichtung (C1, C2) und einem zweidimensionalen Koordinatensystem einer Displayvorrichtung (L) zu bestimmen, eine Segmentierung durchzuführen, um in einem Bild wenigstens Verdeckungen von Hintergrundobjekten durch Vordergrundobjekte zu bestimmen,

wenigstens ein mit dem Analysemodul (A1, A2) verbundenes Kontrollmodul (K),

wenigstens eine Displayvorrichtung (L), die dazu eingerichtet ist, Anzeigeinformation, insbesondere Videoclips, darzustellen, wobei die Bildaufnahmeeinrichtung (C1. C2)dazu eingerichtet ist, die auf der Displayvorrichtung (L) dargestellte Anzeigeinformation aufzunehmen,

eine Datenbank (D), die mit dem Kontrollmodul (K) und der Displayvorrichtung (L) verbunden ist, wobei die Datenbank (D) dazu eingerichtet ist, eine Abfolge von Anzeigeinformationen zu speichern, die nacheinander auf der Displayvorrichtung (L) darzustellen sind,

wobei das Analysemodul (A1, A2) weiter dazu eingerichtet ist, Qualitätsdaten über die Segmentierung zu ermitteln und an das Kontrollmodul (K) zu senden, wobei das Kontrollmodul (K) dazu eingerichtet ist, die auf der Displayvorrichtung (L) dargestellten Anzeigeinformationen basierend auf den Qualitätsdaten anzupassen, **dadurch gekennzeichnet, dass** das Analysemodul (A1, A2) dazu eingerichtet ist, bei der Segmentierung eine Energiefunktion anzuwenden und zu minimieren, wobei die Energiefunktion auf Wahrscheinlichkeitsverteilungen von Vordergrundobjekten und Hintergrundobjekten oder/und auf pixelweisen Wahrscheinlichkeitsverteilungen basiert, und, dass das Kontrollmodul (K) dazu eingerichtet ist, für verbleibende Anzeigeinformationen der Abfolge zu bestimmen, bei welcher Anzeigeinformation sich eine erwartete Wahrscheinlichkeitsverteilung des Vordergrunds und eine erwartete Wahrscheinlichkeitsverteilung des Hintergrund der Bildaufnahmeeinrichtung am meisten unterscheiden.

2. System nach Anspruch 1, **dadurch gekennzeichnet, dass** das Kontrollmodul (K) dazu eingerichtet ist, die gespeicherte Abfolge der Anzeigeinformationen zu verändern.

3. System nach Anspruch 1 oder 2, **dadurch gekennzeichnet, dass** das Kontrollmodul (K) dazu eingerichtet ist, für eine betreffende Bildaufnahmeeinrichtung (C1, C2) eine Wahrscheinlichkeitsabbildung zu berechnen, wobei die Wahrscheinlichkeitsabbildung beschreibt, welche Wahrscheinlichkeitsverteilung in einem Bild der betreffenden Bildaufnahmeeinrichtung (C1. C2) vorliegt, wenn die Displayvorrichtung (L) eine der verbleibenden Anzeigeinformationen darstellt.

4. System nach Anspruch 3, **dadurch gekennzeichnet, dass** das Kontrollmodul (K) dazu eingerichtet ist, die Wahrscheinlichkeitsabbildung basierend auf einer Transformation zwischen dem Koordinatensystem der Displayvorrichtung und dem Koordinatensystem der Bildaufnahmeeinrichtung (C1, C2) zu ermitteln.

5. System nach Anspruch 4, **dadurch gekennzeichnet, dass** das Kontrollmodul (K) dazu eingerichtet ist, die Wahrscheinlichkeitsabbildung basierend auf einer radiometrischen Kalibrierung zwischen der Displayvorrichtung (L) und der betreffenden Bildaufnahmeeinrichtung (C1, C2) zu ermitteln.

6. System nach Anspruch 4 oder 5, **dadurch gekennzeichnet, dass** an der Displayvorrichtung (L) mehrere Kalibriermarken angeordnet sind, die von der Bildaufnahmeeinrichtung (C1, C2) erfassbar sind, um die Transformation zwischen dem Koordinatensystem der Displayvorrichtung (L) und dem Koordinatensystem der Bildaufnahmeeinrichtung (C1, C2) zu erleichtern.

7. System nach einem der vorhergehenden Ansprüche, **dadurch gekennzeichnet, dass** das Kontrollmodul dazu eingerichtet ist, in die Abfolge von Anzeigeinformationen Kalibrierinformation einzufügen, wobei die Displayvorrichtung dazu eingerichtet ist, die Kalibrierinformation darzustellen.

8. System nach Anspruch 7, **dadurch gekennzeichnet, dass** die Kalibrierinformation wenigstens ein einzelnes einfarbiges Bild umfasst, wobei das Bild eine Darstelldauer von wenigstens einem oder genau einem Frame aufweist, und wobei das Bild vorzugsweise eine Farbe aufweist, die einen hohen Kontrast zu einem Vordergrundobjekt aufweist.

9. Verfahren zur dynamischen Kontrastmaximierung zwischen Vordergrund und Hintergrund in Bildern oder/und Bildsequenzen, umfassend die Schritte Erfassen von wenigstens einem Bild mittels wenigstens einer Bildaufnahmeeinrichtung,
Berechnen einer Transformation zwischen einem zweidimensionalen Koordinatensystem der Bildaufnahmeeinrichtung und einem zweidimensionalen Koordinatensystem einer Displayvorrichtung,
Durchführen einer Segmentierung, um in einem Bild wenigstens Verdeckungen von Hintergrundobjekten durch Vordergrundobjekte zu bestimmen,
Darstellen von Anzeigeinformation, insbesondere Videoclips, auf einer Displayvorrichtung, wobei die auf der Displayvorrichtung dargestellte Anzeigeinformation von der Bildaufnahmeeinrichtung aufgenommen wird,

Speichern einer Abfolge von Anzeigeinformationen, die nacheinander auf der Displayvorrichtung (L) darzustellen sind,

wobei bei der Segmentierung Qualitätsdaten über die Segmentierung ermittelt werden und die auf der Displayvorrichtung dargestellten Anzeigeinformationen basierend auf den Qualitätsdaten angepasst werden, **gekennzeichnet durch** Anwenden und Minimieren einer Energiefunktion, wobei die Energiefunktion auf Wahrscheinlichkeitsverteilungen von Vordergrundobjekten und Hintergrundobjekten oder/und auf pixelweisen Wahrscheinlichkeitsverteilungen basiert, und durch Bestimmen für verbleibende Anzeigeinformationen der Abfolge, bei welcher Anzeigeinformation sich eine erwartete Wahrscheinlichkeitsverteilung des Vordergrunds und eine erwartete Wahrscheinlichkeitsverteilung des Hintergrund der Bildaufnahmeeinrichtung am meisten unterscheiden.

**Claims**

1. A system for dynamically maximizing the contrast between foreground and background in images and/or image sequences, comprising

    at least one image-recording device (C1, C2),

    at least one analysis module (A1, A2) connected to the image-recording device (C1, C2), which is configured to determine a transformation between a two-dimensional coordinate system of the image-recording device (C1, C2) and a two-dimensional coordinate system of a display device (L), and carry out a segmentation in order to determine at least occlusions of background objects through foreground objects in an image,

    at least one control module (K) connected to the analysis module (A1, A2),

    at least one display device (L), which is configured to display display information, in particular, video clips, wherein the image-recording device (C1, C2) is configured to record the display information displayed on the display device (L),

    a database (D), which is connected to the control module (K) and to the display device (L), wherein the database (D) is configured to store a sequence of display information to be displayed on the display device (L) one after the other,

    wherein the analysis module (A1, A2) is further configured to identify quality data on the segmentation and transmit same to the control module (K),

    wherein the control module (K) is configured to adjust the display information displayed on the display device (L) based on the quality data,

    **characterized in that**

    the analysis module (A1, A2) is configured to apply and minimize an energy function during segmentation, wherein the energy function is based on probability distributions of foreground objects and background objects and/or on pixel-wise probability distributions,

    and **in that**

    the control module (K) is configured to determine for remaining display information of the sequence, in which display information an expected probability distribution of the foreground and an expected probability distribution of the background of the image-recording device differ the most.

2. The system according to claim 1, **characterized in that** the control module (K) is configured to change the stored sequence of the display information.

3. The system according to claim 1 or 2, **characterized in that** the control module (K) is configured to calculate a probability mapping for a respective image-recording device (C1, C2), wherein the probability mapping describes which probability distribution exists in an image of the respective image-recording device (C1, C2) if the display device (L) displays any one of the remaining pieces of display information.

4. The system according to claim 3, **characterized in that** the control module (K) is configured to determine the probability mapping based on a transformation between the coordinate system of the display device and the coordinate system of the image-recording device (C1, C2).

5. The system according to claim 4, **characterized in that** the control module (K) is configured to determine the probability mapping based on a radiometric calibration between the display device (L) and the respective image-recording device (C1, C2).

6. The system according to claim 4 or 5, **characterized in that** a plurality of calibration marks are arranged on the display device (L), which are detectable by the image-recording device (C1, C2) in order to facilitate the transformation between the coordinate system of the display device (L) and the coordinate system of the image-recording device (C1, C2).

7. The system according to any of the preceding claims, **characterized in that** the control module is configured to insert calibration information into the sequence of display information, wherein the display device is configured to display the calibration information.

8. The system according to claim 7, **characterized in that** the calibration information comprises at least a single monochrome image, wherein the image has a display duration of at least one or precisely one frame, and wherein the image preferably has a color, which has a high contrast to a foreground object.

9. A method for dynamically maximizing the contrast between the foreground and background in images and/or image sequences, comprising the steps of:

detecting at least one image by means of at least one image-recording device,
calculating a transformation between a two-dimensional coordinate system of the image-recording device and a two-dimensional coordinate system of a display device,
carrying out a segmentation in order to determine at least occlusions of background objects through foreground objects in an image,
displaying display information, in particular, video clips, on a display device, wherein the display information displayed on the display device is recorded by the image-recording device,
wherein, during segmentation, quality data on the segmentation are identified, and the display information displayed on the display device are adjusted based on the quality data

**characterized by**

applying and minimizing an energy function, wherein the energy function is based on probability distributions of foreground objects and background objects and/or on pixel-wise probability distributions,
and
determining for remaining display information of the sequence, in which display information an expected probability distribution of the foreground and an expected probability distribution of the background of the image-recording device differ the most.

**Revendications**

1. Système de maximisation dynamique de contraste entre le premier plan et l'arrière-plan dans des images et/ou des séquences d'images, comprenant
au moins un dispositif de prise de vues (C1, C2),
au moins un module d'analyse (A1, A2) relié au dispositif de prise de vues (C1, C2), qui est configuré pour déterminer une transformation entre un système de coordonnées bidimensionnel du dispositif de prise de vues (C1, C2) et un système de coordonnées bidimensionnel d'un équipement d'affichage (L), pour réaliser une segmentation afin de déterminer dans une image au moins des recouvrements d'objets d'arrière-plan par des objets de premier plan,
au moins un module de contrôle (K) relié au module d'analyse (A1, A2), au moins un équipement d'affichage (L) qui est configuré pour représenter une information d'affichage, en particulier des vidéoclips, dans lequel le dispositif de prise de vues (C1, C2) est configuré pour enregistrer l'information d'affichage représentée sur l'équipement d'affichage (L),
une base de données (D), qui est reliée au module de contrôle (K) et à l'équipement d'affichage (L), dans lequel la base de données (D) est configurée pour stocker une séquence d'informations d'affichage qui sont à représenter les unes après les autres sur l'équipement d'affichage (L),
dans lequel le module d'analyse (A1, A2) est configuré par ailleurs pour déterminer des données de qualité portant sur la segmentation et les envoyer au module de contrôle (K),
dans lequel le module de contrôle (K) est configuré pour adapter les informations d'affichage représentées sur l'équipement d'affichage (L) sur la base des données de qualité,
**caractérisé en ce que**
le module d'analyse (A1, A2) est configuré pour appliquer une fonction d'énergie et la minimiser lors de la segmentation, dans lequel la fonction d'énergie repose sur des distributions de probabilité d'objets de premier plan et d'objets d'arrière-plan et/ou sur des distributions de probabilité pixel par pixel,
et que le module de contrôle (K) est configuré pour déterminer pour des informations d'affichage restantes de la séquence l'information d'affichage pour laquelle une distribution de probabilité attendue du premier plan et une distribution de probabilité attendue de l'arrière-plan du dispositif de prise de vues se distinguent le plus.

2. Système selon la revendication 1, **caractérisé en ce que** le module de contrôle (K) est configuré pour modifier la séquence stockée des informations d'affichage.

3. Système selon la revendication 1 ou 2, **caractérisé en ce que** le module de contrôle (K) est configuré pour calculer pour un dispositif de prise de vues (C1, C2) concerné une reproduction de probabilité, dans lequel la reproduction de probabilité décrit la distribution de probabilité qui est présente sur une image du dispositif de prise de vues (C1, C2) concerné quand l'équipement d'affichage (L) représente une des informations d'affichage restantes.

4. Système selon la revendication 3, **caractérisé en ce que** le module de contrôle (K) est configuré pour déterminer la reproduction de probabilité sur la base d'une transformation entre le système de coordonnées de l'équipement d'affichage et le système de coordonnées du dispositif de prise de vues (C1, C2).

5. Système selon la revendication 4, **caractérisé en**

**ce que** le module de contrôle (K) est configuré pour déterminer la reproduction de probabilité sur la base d'un étalonnage radiométrique entre l'équipement d'affichage (L) et le dispositif de prise de vues (C1, C2) concerné.

6. Système selon la revendication 4 ou 5, **caractérisé en ce que** sont disposées sur l'équipement d'affichage (L) plusieurs marques d'étalonnage qui peuvent être détectées par le dispositif de prise de vues (C1, C2) pour faciliter la transformation entre le système de coordonnées de l'équipement d'affichage (L) et le système de coordonnées du dispositif de prise de vues (C1, C2).

7. Système selon l'une des revendications précédentes, **caractérisé en ce que** le module de contrôle est configuré pour insérer dans la séquence d'informations d'affichage une information d'étalonnage, dans lequel l'équipement d'affichage est configuré pour représenter l'information d'étalonnage.

8. Système selon la revendication 7, **caractérisé en ce que** l'information d'étalonnage comprend au moins une unique image à une couleur, dans lequel l'image présente une durée de représentation d'au moins un ou de précisément un cadre, et dans lequel l'image présente de préférence une couleur qui présente un contraste élevé par rapport à un objet de premier plan.

9. Procédé de maximisation dynamique de contraste entre le premier plan et l'arrière-plan dans des images et/ou des séquences d'images, comprenant les étapes suivantes :

   acquérir au moins une image au moyen d'au moins un dispositif de prise de vues,
   calculer une transformation entre un système de coordonnées bidimensionnel du dispositif de prise de vues et un système de coordonnées bidimensionnel d'un équipement d'affichage,
   réaliser une segmentation pour déterminer dans une image au moins des recouvrements d'objets d'arrière-plan par des objets de premier plan,
   représenter une information d'affichage, en particulier de vidéoclips, sur un équipement d'affichage, dans lequel l'information d'affichage représentée sur l'équipement d'affichage est enregistrée par le dispositif de prise de vues,
   stocker une séquence d'informations d'affichage qui sont à représenter les unes après les autres sur l'équipement d'affichage (L),
   dans lequel des données de qualité portant sur la segmentation sont déterminées lors de la segmentation et les informations d'affichage représentées sur l'équipement d'affichage sont adaptées sur la base des données de qualité,
**caractérisé par**
appliquer une fonction d'énergie et la minimiser, dans lequel la fonction d'énergie repose sur des distributions de probabilité d'objets de premier plan et d'objets d'arrière-plan et/ou sur des distributions de probabilité pixel par pixel et déterminer pour des informations d'affichage restantes de la séquence de l'information d'affichage pour laquelle une distribution de probabilité attendue du premier plan et une distribution de probabilité attendue de l'arrière-plan du dispositif de prise de vues se distinguent le plus.

Fig. 1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- GB 2408164 A